(19) Europäisches Patentamt
European Patent Office
Office européen des brevets

(11) Veröffentlichungsnummer: **0 097 912 B2**

(12) **NEUE EUROPÄISCHE PATENTSCHRIFT**

(45) Veröffentlichungstag der neuen Patentschrift: **20.03.91 Patentblatt 91/12**

(51) Int. Cl.$^5$: **C09B 31/11**, // C08K5/23

(21) Anmeldenummer: **83106079.3**

(22) Anmeldetag: **22.06.83**

# (54) Verwendung von Diarylpigmenten zum Pigmentieren von Kunststoffen.

Die Akte enthält technische Angaben, die nach dem Eingang der Anmeldung eingereicht wurden und die nicht in dieser Patentschrift enthalten sind.

(30) Priorität: **26.06.82 DE 3223887**

(43) Veröffentlichungstag der Anmeldung: **11.01.84 Patentblatt 84/02**

(45) Bekanntmachung des Hinweises auf die Patenterteilung: **14.01.87 Patentblatt 87/03**

(45) Bekanntmachung des Hinweises auf die Entscheidung über den Einspruch: **20.03.91 Patentblatt 91/12**

(84) Benannte Vertragsstaaten: **BE CH DE FR GB IT LI**

(56) Entgegenhaltungen:
**CH-A- 400 418**
**FR-E- 90 694**
**GB-A- 1 111 490**
**CHEMICAL ABSTRACTS, Band 93, Nr. 22, Dezember 1980, Seite 83, Nr. 206142b, Columbus, Ohio, USA**
**CHEMICAL ABSTRACTS, Band 89, Nr. 14, 2. Oktober 1978, Seite 159, Nr. 112360m, Columbus, Ohio, USA**

(73) Patentinhaber: **HOECHST AKTIENGESELLSCHAFT**
**Postfach 80 03 20**
**W-6230 Frankfurt am Main 80 (DE)**

(72) Erfinder: **Hunger, Klaus, Dr.**
**Johann-Strauss-Strasse 35**
**W-6233 Kelkheim (Taunus) (DE)**
Erfinder: **Frölich, Heinrich, Dr.**
**Obernhäuser Weg 6**
**W-6272 Niedernhausen/Taunus (DE)**
Erfinder: **Lenz, Hans-Joachim**
**Jahnstrasse 18**
**W-6238 Hofheim am Taunus (DE)**

**EP 0 097 912 B2**

**Beschreibung**

Aus der deutschen Patentschrift 921 404 sind Diarylpigmente bekannt, die durch Kupplung von bis-diazotiertem 3,3′-Dichlor-4,4′-diaminodiphenyl auf Acetessigsäureanilide hergestellt und vorzugsweise in der Druckfarbenindustrie eingesetzt werden ; sie sind wegen ihrer Unlöslichkeit auch für den Einsatz auf dem Lack- und Kunststoffsektor von großer Bedeutung. Im Falle der Pigmentierung von Kunststoffen spielt dabei die Stabilität des Pigmentes gegen Hitzeeinwirkung eine wichtige Rolle. In Abhängigkeit vom zu pigmentierenden Kunststoff werden dabei oft Temperaturen von über 200°C gefordert. Hitzestabilität bedeutet vor allem Farbstärke- und Farbtonkonstanz, d.h. es darf weder Farbvertiefung (durch Lösen oder Zersetzen) noch Farbschwund (z.B. durch Rekristallisation) bei der geforderten Temperatur auftreten.

Diarylpigmente besitzen aber meist keine ausreichende Hitzestabilität und sind daher für manche Kunststoffeinfärbungen, insbesondere für Polyolefine, nur begrenzt einsetzbar. Eine Verbesserung der Hitzestabilität ist daher immer wieder gefordert worden, um diese Pigmente universell einsetzen zu können.

Aufgabe der vorliegenden Erfindung ist somit die Bereitstellung von Diarylpigmenten, die bei der Pigmentierung von Kunststoffen eine gute Hitzestabilität, d.h. eine Stabilität bei Verarbeitungstemperaturen oberhalb 200°C ohne wesentliche Änderung der coloristischen Eigenschaften, insbesondere der Farbstärke aufweisen.

Im Zuge der Bemühungen zur Lösung dieser Aufgabe wurde erfindungsgemäß nunmehr gefunden, daß Diarylpigmente mit ausgezeichneter Hitzestabilität erhalten werden, wenn man während der Azokupplung von bis-diazotiertem 3,3′-Dichlor-4,4′-diaminodiphenyl auf Acetessigsäureanilide zur Herstellung des Pigments geringfügige Zusatzmengen von in 3- bzw. 4-Stellung ein- oder zweifach, durch –NH-CO–Gruppierungen in cyclischer oder offenkettiger Form kernsubstituierten Acetessigsäureaniliden einsetzt.

Gegenstand der Erfindung ist die Verwendung von Diarylpigmenten mit verbesserter Hitzestabilität, welche durch Kupplung von bis-diazotiertem 3,3′-Dichlor-4,4′-diaminophenyl auf ein Acetessigsäureanilid der Formel I

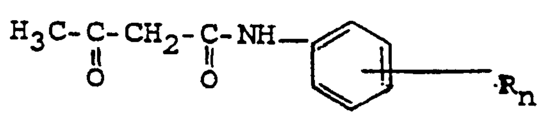

(I),

worin

R gleich oder verschieden ist und ein Wasserstoffatom, ein Chloratom oder eine Methyl-, Methoxy- oder Ethoxygruppe darstellt, und n eine ganze Zahl von 1 bis 3 bedeutet, dadurch erhältlich sind, daß die Azokupplung in Gegenwart von 0, 5 bis 5, 3 Mol-% (bezogen auf das Acetessigsäureanilid der Formel I) einer Verbindung der Formel II

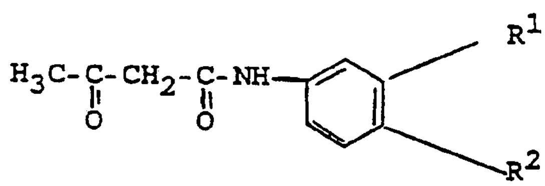

(II),

worin $R^1$ und $R^2$ jeweils einen Rest

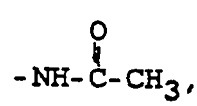

oder einer der Reste $R^1$ und $R^2$ ein Wasserstoffatom und der andere einen Rest

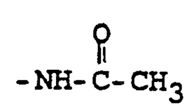

oder

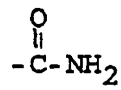

oder

$-SO_2NH_2$,

oder $R^1$ und $R^2$ miteinander verbunden sind und einen zweiwertigen Rest aus der Reihe

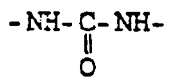 , 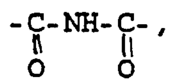 , 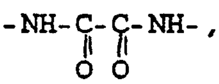 , 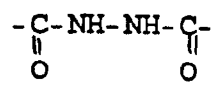 ,

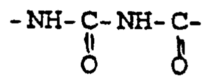

und

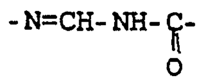

bedeuten, durchgeführt wird, als Farbmittel zum Pigmentieren von Kunststoffen.

Disazoverbindungen der vorstehend erläuterten Gattung, welche durch Kupplung von bis-diazotiertem Dichlordiaminodiphenyl mit Acetoacetaniliden der Formel I in Gegenwart von weiteren Acetoacetaniliden zugänglich sind, werden in der Literatur bereits beschrieben :

So betrifft das Referat in Chem Abstr. 89 (1978) Nr. 112360 m die Erzeugung von Pigmenten durch Kupplung von tetrazotiertem 3,3'-Dichlorbenzidin auf eine Mischung aus Acetoacetylaminobenzol und 1 bis 50 Mol-% Acetoacetylaminobenzamiden oder -benz (alkylamiden). Nach der Lehre dieses Standes der Technik werden durch die Mischkupplung gelbe Druckfarbenpigmente für den Offsetdruck mit ausgezeichneter "heat resistance" erhalten. Wie aus dem erwähnten Beispiel hervorgeht, bezieht sich die "heat resistance" auf den Erhalt der Transparenz (Rekristallisationsstabilität) bei 80 bis 90°C in einem Offsetdruckfirnis im Vergleich zu dem Pigment mit nur einer Kupplungskomponente allein. Die Entgegenhaltung sagt jedoch nichts über die Hitzestabilität des Mischpigments bei Temperaturen über 200°C aus, die bei der Einfärbung von Kunststoffen relevant sind und bei denen neben den quantitativ und qualitativ anderen Bedingungen auch die Stabilität der Pigmentteilchen gegen thermische Zersetzung eine Rolle spielt.

Aus der JP-A-89 356/1980 sind ebenfalls Druckfarbenpigmente bekannt, welche durch Mischkupplung von tetrazotiertem Dichlorbenzidin mit einem Gemisch von Acetoacetarylamiden hergestellt werden und die eine verbesserte Transparenz beim Einsatz auf dem besagten Anwendungssektor haben. Auch hier fehlt wiederum jeglicher Hinweis auf eine verbesserte Hitzestabilität der erwähnten Mischpigmente.

Die FR-E-90 694 ist schließlich mit Mischkupplungspigmenten aus tetrazotiertem Dichlorbenzidin und zwei verschiedenen Acetoacetarylamiden befaßt, wobei in der Mischung der Kupplungskomponenten ein Verhältnis von 5-95 : 95-5 Mol-% vorliegen kann. Während die Entgegenhaltung auf Pigmente mit verbesserter Rheologie, hoher Farbstärke und verbesserter Transparenz gerichtet ist, fehlen Angaben, die auf eine gute oder verbesserte Hitzestabilität schließen lassen.

Wie aufgrund von experimentellen Untersuchungen mit den anhand der offenbarten Beispiele in der FR-E-90 694 strukturell nächstliegenden Disazopigmenten hervorgeht, wurden im Ergebnis für die Mischkupplungspigmente der Entgegenhaltung keine Verbesserung, sondern teilweise sogar eine Verschlechterung der Hitzestabilität in Polyethylen gefunden. Die erfindungsgemäßen Pigmente mit den verbesserten Hitzestabilitäten sind somit nicht nahegelegt.

Für zuvor erläuterten speziellen Verwendungszwecke zur Pigmentierung von Kunststoffen werden vorzugsweise Diarylpigmente des oben bezeichneten Typs eingesetzt, in denen der Anteil der Verbindung der Formel II 1 bis 3 Mol-% (bezogen auf die Menge an Acetessigsäureanilid der Formel I) beträgt.

Als Verbindungen der Formel II kommen bevorzugt solche in Betracht, bei denen $R^1$ ein Wasserstoffatom

und $R^2$ einen Rest

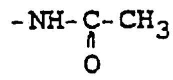

oder

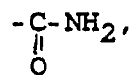

oder umgekehrt bedeutet, oder $R^1$ und $R^2$ miteinander verbunden einen Rest der Reihe

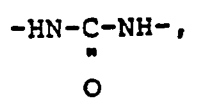 , 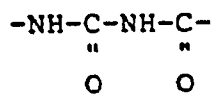 oder 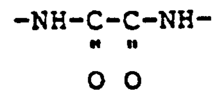

darstellt, z.B. 6- oder 7-Acetoacetylamino-tetrahydro-chinazolindion-2,4, 6-Acetoacetylamino-tetrahydro-chinoxalindion-2,3 ; besonders bevorzugt ist 5-Aceto-acetylamino-benzimidazolon.

Die als Kupplungskomponente verwendeten Acetessigsäureanilide der Formel 1 sind vorzugsweise Acetessigsäure-2-methylanilid, -2-methoxyanilid, 4-ethoxyanilid, -2,4-dimethylanilid, -2-methyl-4-chloranilid, -2-methoxy-5-chloranilid, -2,4-dimethoxy-5-chloranilid, insbesondere jedoch Acetessigsäure -2,5-dimethoxy-4-chloranilid.

Die Diazotierung des 3,3'-Dichlor-4, 4'-diaminodiphenyls erfolgt in bekannter Weise mit Alkalinitriten oder niederen Alkylnitriten mit ausreichend starken Säuren, insbesondere mit Mineralsäuren z.B. Salzsäure oder Schwefelsäure, kann aber auch mit Nitrosylschwefelsäure durchgeführt werden. Es kann hierbei wie auch bei der anschließenden Kupplung von Nutzen sein, einen Zusatz von oberflächenaktiven Mitteln zu verwenden, zum Beispiel nichtionogenen, anionaktiven oder kationaktiven Dispergiermitteln.

Solche oberflächenaktive Mittel sind z.B. anionaktive Substanzen wie Fettsäuretauride, Fettsäure-N-methyltauride, Fettsäureisäthionate, Alkylbenzolsulfonate, Alkylnaphthalinsulfonate, Alkylphenolpolyglykolethersulfate und Fettalkoholpolyglykolethersulfate, Fettsäuren, z.B. Palmitin-, Stearin- und Ölsäure, Seifen z.B. Alkalisalze von Fettsäuren, Naphthensäuren und Harzsäuren, z.B. Abietinsäure, alkalilösliche Harze, z.B. kolophoniummodifizierte Maleinatharze, kationaktive Substanzen, wie quaternäre Ammoniumsalze, N-oxide tertiäre Amine bzw. ihrer Salze, Fettamine und ihre oxethylierten Derivate, nichtionogene Substanzen wie Fettalkoholpolyglykolether (ethoxylierte Fettalkohole), Fettsäurepolyglykolester, Alkylphenolpolyglykolether und Dialkylpolyglykolether.

Die oberflächenaktiven Mittel können einzeln oder auch als Gemische zugesetzt werden. Die Menge kann in weiten Grenzen schwanken, wobei im allgemeinen 0,1 bis 20 Gew.-%, vorzugsweise 5 bis 10 Gew.-%, bezogen auf das Pigment eingesetzt werden.

Vor der Kupplung geht man zweckmäßigerweise so vor, daß man eine Verbindung der allgemeinen Formel II mit der Kupplungskomponente mischt, anschließend diese Mischung löst, vorzugsweise in wäßriger Alkalilauge, schließlich mit Säure wieder ausfällt und mit der erhaltenen Suspension die Kupplung durchführt.

Diazotierung und Kupplung können auch in Gegenwart geeigneter organischer Lösungsmittel durchgeführt werden, zum Beispiel Eisessig, niederen Alkanolen, Dioxan, Formamid, Dimethylformamid, Dimethylsulfoxid, Pyridin oder N-Methylpyrrolidon.

Zur Erzielung der vollen Farbstärke und einer besonders günstigen Kristellstruktur ist es oft zweckmäßig, nach der Kupplung das Reaktionsgemisch beispielsweise einige Zeit bis auf Rücktlußtemperatur zu erhitzen oder unter Druck bei Temperaturen oberhalb 100°C zu halten, gegebenenfalls in Gegenwart von organischen Lösungsmitteln oder in Gegenwart von Harzseifen und/oder anderen löslichen Harzen. Besonders rein und gut dispergierbare Pigmente erhält men mit den erfindungsgemäßen Produkten, wenn man nach der Kupplung die feuchten Preßkuchen oder die getrockneten Pulver der angeführten thermischen Nachbehandlung, gegebenenfalls mit organischen Lösungsmitteln, unterwirft oder eine Mahlung der Pigmente unter Zusatz von Mahlhiltsmitteln anschließt.

Die Herstellung der Pigmente kann auch in Gegenwart eines Trägermaterials, beispielsweise von Schwerspat, erfolgen.

Die erfindungsgemäß eingesetzten Verbindungen sind unlösich Wasser und in den üblichen organischen

Lösungsmitteln und eignen sich hervorragend für die Pigmentierung von Kunststoffen, z. B. von Polyolefinen, deren Verarbeitungstemperaturen oberhalb 200°C liegen, sowie von Polyvinylchlorid, Polyurethanen, Polyacrylnitril oder Polydioldicarboxylaten, z. B. Polyglykolterephtalaten.

Die Pigmente lassen sich in den genannten Medien gut verarbeiten. Die pigmentierten Substrate weisen sehr gute Lichtechtheiten und insbesondere eine hervorragende Hitzestabilität auf und sind gegen den Einfluß von Chemikalien, vor allem Lösungsmitteln, beständig.

In den nachfolgenden Beispielen beziehen sich Teile und Prozentangaben auf das Gewicht.

Beispiele

1 a) 50,6 Teile 3,3'-Dichlor-4,4'-diamino-diphenyl wurden in 350 Volumenteilen Wasser und 120 Volumenteilen 31%iger Salzsäure angerührt und dann in Gegenwart von 1,7 Teilen eines Aluminium-Hydrosilikats bei 0-5°C durch schnelle Zugabe von 4,6 Volumentalen 40%iger Natriumnitrilösung bisdiazotiert. Es wurde nachgerührt und der Nitritüberschuß größenteils zerstört. Die erhaltene Bisdiazoniumsalzlösung wurde geklärt und jeweils zur Hälfte für die folgende Azokupplung und für die Azokupplung im Vergleichsbeispiel 1 b verwendet.

55,5 Teile 1-Acetoacetylamino-2,5-dimethoxy-4-chlor-benzol und 0,48 Teile 5-Acetoacetylamino-benzimidazolon wurden in 200 Volumenteilen Wasser und 22 Volumenteilen 33%iger Natronlauge gemeinsam gelöst und unter Rühren bei etwa 15°C zu einer Mischung, die aus etwa 200 Volumenteilen Wasser, 18 Volumenteilen Eisessig und 10 Volumenteilen einer 10%igen wäßrigen Lösung eines handelsüblichen ethoxylierten Stearylalkohols bestand zugegeben und dabei ausgefällt. Zu der erhaltenen Fällung wurde dann eine Lösung von 2,7 Teilen eines handelsüblichen N-Dimethyl-Cocosfett-aminoxids (der Cocosfett-Rest steht für ein Gemisch von Alkylresten mit folgender Kettenverteilung. $C_8$ 6%, $C_{10}$6%, $C_{12}$ 54%, $C_{14}$ 18%. $C^{18}/C^{18}$ unges. 8%) in 6 Volumenteilen Wasser zugesetzt. Zur Kupplung ließ man die geklärte Bisdiazoniumlösung bei etwa 20-25°C innerhalb von 3 Stunden zur vorgelegten Suspension der Kupplungs- und Zusatzkomponente zutropfen, wobeinach Erreichen von pH 4,8 dieser pH-Wert durch gleichzeitige Zugabe verdünnter Natronlauge gehalten wurde. Nach beendeter Kupplung wurde zunächst auf 50°C erwärmt, die Suspension des pigments mit ca. 25 Volumenteilen 33%iger Natronlauge alkalisch gestellt, 2,5 Teile Natriumsulfit – es kann auch eine entsprechende Menge Natriumdisultit zugegeben werden – zugesetzt und weiter auf 96-98°C hochgeheizt. Nach einer Stunde bei dieser Temperatur wurde durch Zugabe von Wasser auf 70°C abgekühlt und das Pigment als Preßkuchen isoliert, mit Wasser gewaschen, getrocknet und zu einem Pigmentpulver von rotstichig gelbern Farbton vermahlen.

1 b) (Vergleich) Beispiel 1 a) wurde mit der Abänderung wiederholt, daß als Kupplungskomponente nur 56,06 Teile 1-Acetoacetylamino-2,5-dimethoxy-4-chlor-benzol eingesetzt wurden.

1 c) Prüfung in High Density (HD)-Polyethylen :

I. Das nach Beispiel 1 a) erhaltene Pigmentpulver wurde mit HD-Polyethylen-Pulver Dichte (nach DIN 53479 : 0, 955-0, 959, MFI nach DIN 53735 : 190/2, 16...8 g/10 min, 190/5... 20-26 g/10 min) nach standardisiertem Verfahren vermischt und unter Zusatz von 1% Titandioxid in Form einer Weißpräparation in Granulatform nach DIN 53235 bei aufsteigenden Temperaturen zu Spritzgieß-Prüfkörpern der Standardfarbtiefe ST 1/3 verarbeitet. Pigment 1 a) zeigte eine deutlich bessere Stabilität bei der thermischen Belastung bei Temperaturen von 160°C bis 260°C als das entsprechend verarbeitete Vergleichspigment 1 b): Bezogen auf die probe 1 a) bei 180°C ($\Delta$ E = 0) wurde der Farbabstand $\Delta$ E = 3 nach DIN 6174 (CIELAB-Formel) für eine Belastungsdauer von 1 Minute bzw. 5 Minuten bei 260°C noch nicht erreicht (d.h. bei dieser Toleranzgrenze ist die Hitzestabilität > 260°C), während das Vergleichspigment 1 b) den Farbabstand $\Delta$ E = 3 bereits bei 220° bzw. 210°C erreichte (d.h. seine Hitzestabiltät ist nur 210-220°C). Gleichzeitig blieb beim Pigment 1 a) die Farbstärke bis 260°C annähernd konstant, während sie beim Vergleichspigment 1 b) bis zu dieser Temperatur um etwa 30-40% abnahm.

II. Wurden das Pigment 1 a) und das Vergleichspigment 1 b) vor der Einfärbung in das HD-Polyäthylen zu einer 40% igen polyethylen-Wachs-Präparation verarbeitet, so ergeben sich gleiche Werte bezüglich der thermischen Stabilität wie bei Prüfung 1.

III. Die thermische Stabilität des Pigments 1 a) wer auch in Polypropylen (Dichtenach DIN 53479 : 0,905, MFI nach DIN 53735 : 190/5...3 g/10 min, 230/2...1,5 g/10 min, 230/5...7 g/10 min) bei Temperaturen von 180°C bis 280°C besser als die des Vergleichspigments 1 b). Während der Farbabstand $\Delta$ E = 3 bei Prüfkörpern aus Polypropylen, die analog I hergestellt wurden, bei einer Belastungsdauer von 1 Minuten mit Pigment 1 a) bei 280°C noch nicht erreicht wurde, zeigte sich dieser Wert beim Vergleichspigment 1 b) bereits bei 230°C.

2 a) 101,2 Teile 3,3'-Dichlor-4,4'-diamino-diphenyl wurden analog Beispiel 1 bisdiazotiert. 168,76 Teile 1-Aceto-acetylamino-2,4-dimethyl-benzol und 3,91 Teile 5, Aceto-acetylamino-benzimidazolon wurden in 2000 Volumenteilen Wasser und 80 Volumenteilen 33%iger Natronlauge gemeinsam gelöst und darauf bei

etwa 10°C mit 57,1 Volumenteilen Eisessig wieder ausgefällt und die Fällung mit verdünnter Natronlauge auf pH 5,7 gestellt.

Zur Kupplung ließ men die geklärte Bisdiazoniumsalzlösung bei etwa 20°C in etwa 2 Stunden zum vorgelegten Gemisch der Kupplungs- und Zusatzkomponente zutropfen, wobei nach Erreichen von pH 4,5 dieser pH-Wert durch gleichzeitige Zugabe verdünnter Natronlauge gehalten wurde. Nach beendeter Kupplung wurde hochgeheizt und 1 Stunde bei 96-98°C gehalten, darauf mit Wasser auf 70°C abgekühlt, das Pigment als Preßkuchen isoliert, mit Wasser gewaschen, getrocknet und zu einem Pigment von rotstichig gelbem Farbton vermahlen.

2 b) (Vergleich) Beispiel 2 a wurde mit der Abänderung wiederholt, daß als Kupplungskomponente nur 172,2 Teile 1-Acetoacetylamino-2,4-dimethyl-benzol eingesetzt wurden.

2 c) Bei Prüfung des nach Beispiel 2 a) erhaltenen Pigmentpulvers in HD-Polyethylen (wie unter Beispieil 1 c, I beschrieben) zeigte dieses Pigment ebenfalls eine deutlich verbesserte Stabilität bei thermischer Belastung als Pigment 2 b).

| | Pigment 2 a | Pigment 2 b (Vergleich) |
|---|---|---|
| Hitzestabilität (Belastungsdauer 1 min) | > 260°C | bis 220°C |

3 a) 101,2 Teile 3,3'-Dichlor-4,4', diamino-diphenyl wurden wie in Beispiel 1 a beschrieben bisdiazotiert und mit einer analog gefällten Suspension aus 236,53 Teilen 1-Aceto-ecetylamino-2,5,dimethoxy-4-chlorbenzol und 2,05 Teilen 5-Acetoacetylamino, benzimidazolon ohne Zusatz des N-Dimethyl-Fettaminoxids gekuppelt. Nach beendeter Kupplung wurde eine Lösung von 73,3 Teilen einer handelsüblichen etwa 50%igen Harzseife zugegeben, für 15 Minuten bei einer Temperatur von 85°C gehalten und weiter wie in Beispiel beschrieben behandelt. Es wurde ein Pigment mit ähnlich guten Eigenschaften wie im Beispiel 1 a) beschreben erhalten.

3 b) (Vergleich) Beispiel 3 a) wurde mit der Abänderung wiederholt, daß als Kupplungskomponente nur 236,9 Teile 1-Aceto-acetylamino-2,5-dimethoxy-4-chlor-benzol eingesetzt wurden.

3 c) Bei Prüfung des nach Beispiel 3 a) erhaltenen Pigmentpulvers in HD-Polyethylen (wie unter Beispiel 1 c, I, beschrieben) zeigte dieses Pigment ebenfalls eine deutliche verbesserte Stabilität bei thermischer Belastung : größer als 260°C bei 1-minütiger und 5-minütiger Belastung, während das Vergleichspigment unter diesen Bedingungen nur bis 230°C stabil war.

| | | Pigment 3 a | Pigment 3 b (Vergleich) |
|---|---|---|---|
| Hitzestabilität | | | |
| Belastungsdauer | 1 min | > 260°C | bis 230°C |
| " | 5 min | > 260°C | bis 230°C |

4 bis 20) In analogen Verfahren wurden 25,3 Teile 3,3,-Dichlor-4,4'-diamino-diphenyl bisdiazotiert und mit den in der folgenden Tabelle angegebenen Mengen an Kupplungskomponenten und Zusätzen umgesetzt. Die erhaltenen Pigmente 4 a) - 20 a) wiesen ebenfalls verbesserte Hitzestabilitäten bei der Einfärbung in HD-Polyethylen (gemäß Prüfung II Beispiel 1 c) auf, verglichon mit den entsprechenden Pigmenten 4 b) - 20 b), die ohne Verwendung des in der Tabelle angegebenen Zusatzes hergesellt wurden.

**Tabelle**

| Beispiel | Kupplungskomponente 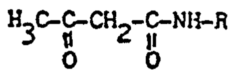 | | Zusatz 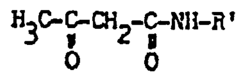 | | Farbton |
|---|---|---|---|---|---|
| | R | Menge (Tle) | R' | Menge (Tle) | |
| 4 a | 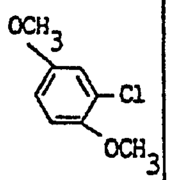 | 54,94 | 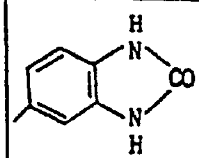 | 0,96 | rotstichig gelb |
| 5 a | " | 54,38 | " | 1,44 | " |
| 6 a | 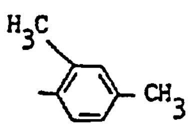 | 41,54 | " | 1,71 | " |
| 7 a | " | 40,90 | " | 2,45 | " |
| 8 a | 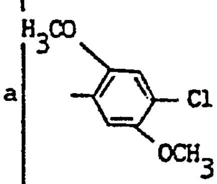 | 54,66 | 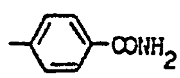 | 1,14 | " |
| 9 a | " | 54,66 | 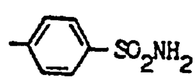 | 1,32 | " |
| 10 a | 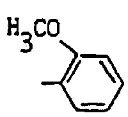 | 42,38 | 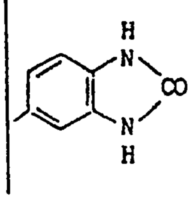 | 1,22 | grünstichig gelb |

**Fortsetzung der Tabelle**

| | | | | | |
|---|---|---|---|---|---|
| 11 a | 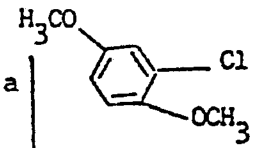 | 54,66 | 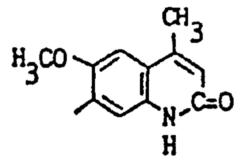 | 1,48 | rotstichig gelb |
| 12 a | 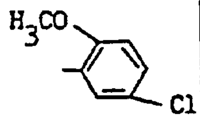 | 49,37 | 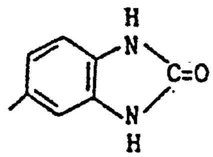 | 0,48 | " |
| 13 a | " | 48,87 | " | 0,97 | " |
| 14 a | 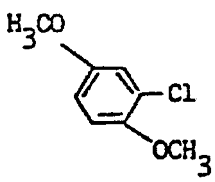 | 54,66 | 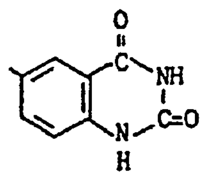 | 1,35 | rotstichig gelb |
| 15 a | " | " | 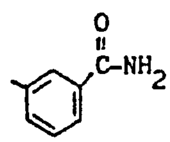 | 1,14 | " |
| 16 a | " | " | 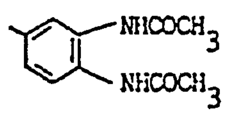 | 1,5 | " |
| 17 a | " | " | 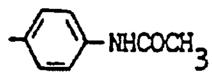 | 1,2 | " |
| 18 a | " | " | 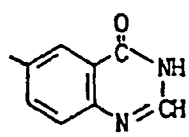 | 1,27 | " |
| 19 a | " | 55,5 | 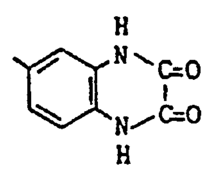 | 0,54 | " |
| 20 a | " | 54,66 | 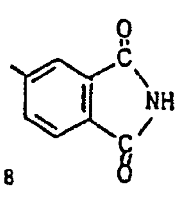 | 1,27 | " |

## Ansprüche

1. Verwendung von Diarylpigmenten, welche durch Kupplung von bis-diazotiertem 3,3'-Dichlor-4,4'-diaminodiphenyl auf ein Acetessigsäureanilid der Formel I

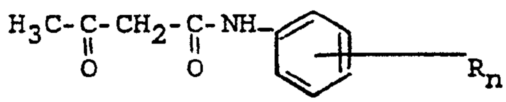

(I),

worin
R gleich oder verschieden ist und ein Wasserstoffatom, ein Chloratom oder eine Methyl-, Methoxy- oder Ethoxygruppe darstellt, und n eine ganze Zahl von 1 bis 3 bedeutet, dadurch erhältlich sind, daß die Azokupplung in Gegenwart von 0,5 bis 5,3 Mol-% (bezogen auf das Acetessigsäureanilid der Formel I) einer Verbindung der Formel II

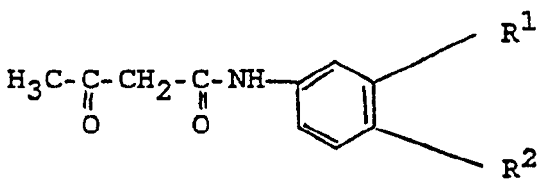

(II),

worin $R^1$ und $R^2$ jeweils einen Rest

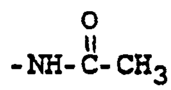,

oder einer der Reste $R^1$ und $R^2$ ein Wasserstoffatom und der andere einen Rest

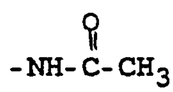

oder

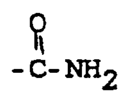

oder

$-SO_2NH_2$,

oder $R^1$ und $R^2$ miteinander verbunden sind und einen zweiwertigen Rest aus der Reihe

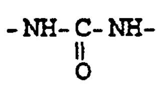, 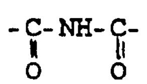, 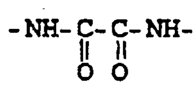, 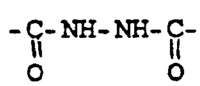,

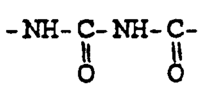 und 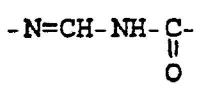

bedeuten, durchgeführt wird, als Farbmittel zum Pigmentieren von Kunststoffen.

2. Verwendung von Diarylpigmenten gemäß Anspruch 1, bei denen die Kupplung in Gegenwart von 1 bis 3 Mol-% (bezogen auf das Acetessigsäureanilid der Formel I) der Verbindung der Formel II durchgefürt wird.

3. Verwendung von Diarylpigmenten gemäß Anspruch 1 oder 2, bei denen als Verbindung der Formel II 5-Acetoacetylaminobenzimidazolon-(2) eingesetzt wird.

4. Verwendung von Diarylpigmenten gemäß Anspruch 1, 2 oder 3, bei denen als Acetessigsäureanilid der Formel I 1-Acetoacetylamino-2,5-dimethoxy-4-chlorbenzol eingesetzt wird.

## Claims

1. Use of diaryl pigments obtainable by coupling of bis-diazotized (3,3'-dichlor- 4,4'-diaminodiphenyl) onto an acetoacetanilide of the formula I

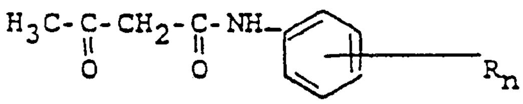

(I),

in which R is identical or different and is a hydrogen atom, a chlorine atom or a methyl, methoxy or ethoxy group, and n is an integer from 1 to 3, by carrying out the azo coupling reaction in the presence of 0.5 to 5.3 mol% (relative to the acetoacetanilide of the formula I) of a compound of the formula II

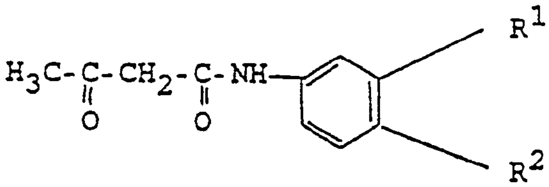

(II),

in which $R^1$ and $R^2$ are each a radical 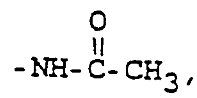

or one of the radicals $R^1$ and $R^2$ is a hydrogen atom and the other is a radical

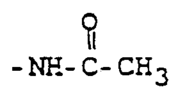

or

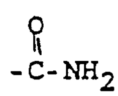

or $-SO_2NH_2$, or $R^1$ and $R^2$ are linked to one another and are a divalent radical from the series comprising

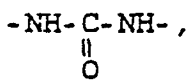 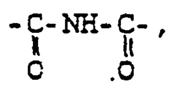 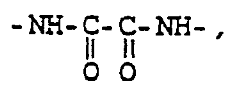 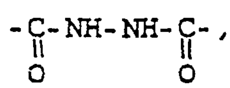

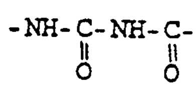

and

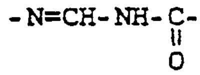

as colorant for pigmenting plastic.

2. Use of diaryl pigments as claimed in claim 1, in which the coupling reaction is carried out in the presence of 1 to 3 mol% (relative to the acetoacetanilide of the formula I) of the compound of the formula II.

3. Use of diaryl pigments as claimed in claim 1 or 2, in which 5-acetoacetylamino-2-benzimidazalone is used as the compound of the formula II.

4. Use of diaryl pigments according to claim 1, 2 or 3, in which 1-acetoacetylamino-2,5-dimethoxy-4-chlorobenzene is used as the acetoacetanilide of the formula I.

## Revendications

1. Application de pigments diaryliques qui peuvent être obtenus par copulation du dichloro-3,3′ diamino-4,4′ biphényle bis-diazoté sur un acétoacétanilide répondant à la formule I :

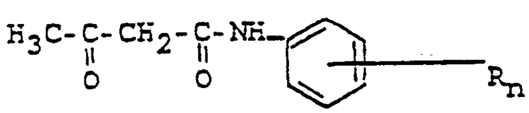
(I),

dans laquelle les R représentent chacun, indépendemment l'un de l'autre, un atome d'hydrogène ou de chlore ou un radical méthyle, méthoxy ou éthoxy et n désigne un nombre entier de 1 à 3, ladite copulation étant effectuée en présence de 0,5 à 5,3% en moles, par rapport à l'acétoacétanilide de formule I, d'un composé répondant à la formule II :

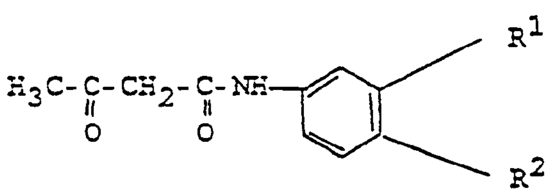
(II),

dans laquelle $R^1$ et $R^2$ représentent chacun un radical

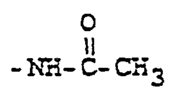
,

ou l'un des radicaux $R^1$ et $R^2$ représente un atome d'hydrogène et l'autre un radical

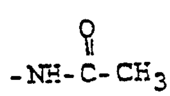

ou

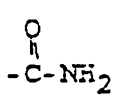

ou $-SO_2NH_2$, ou encore $R^1$ et $R^2$ forment, unis l'un à l'autre, un radical bivalent pris parmis les suivants :

$$-NH-\underset{\underset{O}{\|}}{C}-NH-,\quad -\underset{\underset{O}{\|}}{C}-NH-\underset{\underset{O}{\|}}{C}-,\quad -NH-\underset{\underset{O}{\|}}{C}-\underset{\underset{O}{\|}}{C}-NH-,\quad -\underset{\underset{O}{\|}}{C}-NH-NH-\underset{\underset{O}{\|}}{C}-,$$

$$-NH-\underset{\underset{O}{\|}}{C}-NH-\underset{\underset{O}{\|}}{C}-\quad \text{et} \quad -N=CH-NH-\underset{\underset{O}{\|}}{C}-$$

Comme colorants pour la pigmentation de matières plastiques.

2. Application de pigments diaryliques selon la revendication 1, caractérisée en ce que la copulation est effectuée en présence de 1 à 3% en moles, par rapport à l'acétoacétanilide de formule I, du composé de formule II.

3. Application de pigments diaryliques selon l'une des revendications 1 et 2, caractérisée en ce qu'on utilise, comme composé de formule II, l'acétoacétylamino-5 benzimidazolone-2.

4. Application de pigments diaryliques selon l'une quelconque des revendications 1, 2 et 3, caractérisée en ce qu'on utilise, comme acétoacétanilide de formule I, l'acétoacétyl-amino-1 diméthoxy-2,5 chloro-4 benzène.